# EUROPEAN PATENT APPLICATION

(11) **EP 2 123 295 A1**
(43) Date of publication of application: **25.11.2009**
(21) Application number: 07850870.2
(22) Date of filing: 19.12.2007
(51) Int. Cl.: A61K 38/45, A61K 9/08, A61K 31/573, A61K 45/00, A61K 47/24, A61K 47/48, A61P 11/00, A61P 29/00

(54) **THERAPEUTIC AGENT FOR INTERSTITIAL PNEUMONIA**

(30) Priority: 19.12.2006 JP 2006340845; 05.04.2007 JP 2007099201
(71) Applicant: LTT Bio-Pharma Co., Ltd., Minato-ku, Tokyo 1048315 (JP)
(72) Inventor: SHIRAI, Kouji, Sakura-shi Chiba 285-8741 (JP); KAWASHIMA, Tatsuo, Sakura-shi Chiba 285-8741 (JP); KURODA, Toshihisa, Sakura-shi Chiba 285-8741 (JP); MIZUSHIMA, Yutaka, deceased (JP); MURAKAMI, Masahiro, Kitasaku-gun Nagano 389-0103 (JP); FUKUZAKI, Tomoharu, Ichikawa-shi Chiba 272-0138 (JP)
(74) Representative: Albrecht, Thomas
(86) International application number: PCT/JP2007/074400
(87) International publication number: WO 2008/075706

(57) **Abstract**

A therapeutic agent for interstitial pneumonia is provided which effectively exploits the effect of superoxide dismutase (SOD). The therapeutic composition for interstitial pneumonia contains 10 to 100mg of lecithinized superoxide dismutase represented by the following general formula (I):

SOD'(Q-B)ₘ (I)

(wherein SOD' is a residue of superoxide dismutase; Q is a chemical crosslink; B is a residue of lysolecithin having the hydrogen atom of the hydroxyl group at position 2 of its glycerol moiety removed; and m is the average number of lysolecithin molecules bound to one molecule of the superoxide dismutase and is an integer of 1 or greater) and further contains sucrose to give it a stable form suitable for intravenous administration.

## Description

### TECHNICAL FIELD

The present invention relates to a therapeutic composition for interstitial pneumonia and, in particular, to a therapeutic composition for interstitial pneumonia containing a lecithinized superoxide dismutase (which may be referred to simply as "PC-SOD," hereinafter) as an active ingredient.

### BACKGROUND ART

Superoxide dismutases (which may be referred to simply as "SODs," hereinafter) are physiologically active proteins that exhibit anti-inflammatory activity and were first extracted by Huber et al in 1965 from cow's blood. It has been shown that these proteins can specifically eliminate superoxide anions (O₂⁻), one of active oxygen species that are released in a living body primarily by phagocytic cells such as neutrophiles and macrophages to kill bacteria. SODs and various other antioxidative substances generally exist to protect healthy cells against damage from excessive active oxygen.

When, however, active oxygen is present in amounts that exceed the antioxidative ability of SODs and other antioxidative substances, it attacks any matters that exist nearby, in particular, the cell membrane, causing various disease conditions. In fact, active oxygen with its notable cytotoxic activity has been revealed to be an important factor that causes or enhances various disease conditions, ranging from inflammation and allergies, to tissue damage caused by ischemic reperfusion, and to pulmonary fibrosis following anticancer drug treatment.

Under such circumstances, an SOD that has the ability to specifically eliminate active oxygen has been found and the possibility of its clinical application has been widely investigated. In our extensive investigation of clinical applicability of SODs, the present inventors have come to conclude that in order to enhance the clinical effect of SODs, it is important to maintain the blood levels of SODs by reducing their excretion by the kidney, as well as to eliminate excessive active oxygen present on the cell membrane by increasing the affinity of SODs for the cell membrane. The present inventors have thus studied various modified SODs and proposed lecithinized superoxide dismutase (PC-SOD) (Patent Document 1 and Patent Document 9).

The PC-SOD, or lecithinized superoxide dismutase, is prepared by first preparing Cu/Zn-human superoxide dismutase (SOD) by gene recombination techniques, and then chemically binding an average of four molecules of a lecithin derivative (phosphatidylcholine derivative: PC) to one molecule of SOD (dimer). PC-SOD has high affinity for the cell membrane and has been demonstrated to be highly effective against disease conditions in which active oxygen plays an active role in the affected regions, such as ischemic/reperfusion injury and cardiomyopathy induced by anthracycline-based anticancer drugs. Various drugs containing PC-SOD as an active ingredient have been proposed thus far, including therapeutic agent for acute heart failure (Patent Document 2), antiviral agent (Patent Document 3), therapeutic agent for lupus nephritis (Patent Document 4), treatment for cerebral vascular accident-related dysfunction (Patent Document 5), anti-fibrosis agent (Patent Document 6), treatment for allergic diseases (Patent Document 7) and therapeutic agent for burns (Patent Document 8).

Pneumonia is an infectious disease in which pathogens invade and grow in the alveoli, inducing biological responses. In comparison, interstitial pneumonia is a disease primarily affecting the alveolar wall (characterized primarily by the thickening, cell infiltration and fibrosis) to cause protracted inflammation and fibrosis. Interstitial pneumonia can be classified into different types depending on whether or not their etiology is known. It is an intractable disease in which inflammation results in an increased volume of cells and collagen, thickening of the alveolar wall, reduced oxygen uptake and, thus, shortness of breath (dyspnea).
Although some cases of interstitial pneumonia are transient, the hardening of lung tissue progresses slowly and irreversibly in most cases, ultimately leading to pneumatic fibrosis in which the lung tissue is stiffened to a degree that breathing is no longer possible.

Of different terms that describe different types of interstitial pneumonias, idiopathic interstitial pneumonia (IIP) is a collective term that refers to a group of interstitial pneumonias of unknown etiology, rather than a single disease. IIP is generally a synonym for idiopathic pulmonary fibrosis (IPF) used in the US. The Japanese version of the term was coined in 1981 by a team of Japanese researchers studying interstitial pneumonias. The symptoms of IIP progress slowly in most patients while some patients may experience rapid progress. In either case, the prognosis will be poor, often resulting in a high fatality rate.
In Japan, idiopathic interstitial pneumonias are classified into the following distinct clinicopathological entities: idiopathic pneumatic fibrosis (IPF), nonspecific interstitial pneumonia (NSIP), acute interstitial pneumonia (AIP), cryptogenic organizing pneumonia (COP), respiratory bronchiolitis-associated interstitial lung disease (RB-ILD), desquamative interstitial pneumonia (DIP) and lymphocytic interstitial pneumonia (LIP).

The causes of idiopathic interstitial pneumonias still remain unknown: inflammations and immune responses are suspected to be involved in the fibrosis of the lung, as are various genetic backgrounds.

Recently, it was discovered that interstitial pneumonia can occur as a side effect of anticancer drugs. In particular, interstitial pneumonia is now recognized as one of the serious side effects associated with the use of gefitinib.
Interstitial pneumonia is known to be caused not only by the use of anticancer drugs, but also by the use of Chinese herbal medicines, antirheumatic drugs, antibiotics or interferons. The disease is also known to be associated with collagen diseases. Furthermore, the correlation of interstitial pneumonia with severe acute respiratory syndrome (SARS) or new influenza viruses is suggested.

In each case, the induction of cytotoxic effects or allergy reactions following the onset of interstitial pneumonia involves superoxide anions and other active oxygen species and iron complexes. Thus, such induction may be prevented and, as a consequence, interstitial pneumonia may be treated by eliminating active oxygen species by SOD or similar enzymes.
From this point of view, the present inventors have attempted to treat patients with interstitial pneumonia, in particular, those with idiopathic interstitial pneumonia, by using the previously proposed lecithinized superoxide dismutase (PC-SOD) having high affinity for the cell membrane. As it turned out, PC-SOD is highly effective in the treatment of interstitial pneumonia and becomes particularly stable when combined with a stabilizing agent, in particular, sucrose. This finding ultimately led to the present invention.

The specific concept of using PC-SOD itself in the treatment of interstitial pneumonia, especially idiopathic interstitial pneumonia, has never existed before. Thus, the present invention is truly unique.
Patent Document 1: Japanese Patent Application Laid-Open No. Hei 9-117279
Patent Document 2: Japanese Patent Application Laid-Open No. Hei 9-52843
Patent Document 3: Japanese Patent Application Laid-Open No. Hei 9-59178
Patent Document 4: Japanese Patent Application Laid-Open No. Hei 9-110717
Patent Document 5: Japanese Patent Application Laid-Open No. Hei 10-338645
Patent Document 6: Japanese Patent Application Laid-Open No. 2001-2585
Patent Document 7: Japanese Patent Application Laid-Open No. 2001-151695
Patent Document 8: Japanese Patent Application Laid-Open No. 2006-169128
Patent Document 9: Japanese Patent Application Laid-Open No. 2001-64199

### DISCLOSURE OF THE INVENTION

### PROBLEMS TO BE SOLVED BY THE INVENTION

In view of the above-described present state of the art, it is an object of the present invention to provide a therapeutic composition for interstitial pneumonia containing PC-SOD as an active ingredient. In particular, the object of the present invention is to provide a therapeutic composition for interstitial pneumonia, especially idiopathic interstitial pneumonia, that contains PC-SOD as an active ingredient, along with sucrose as a stabilizing agent for stabilizing PC-SOD.

### MEANS FOR SOLVING THE PROBLEM

To achieve the above-described objects, one essential aspect of the present invention provides a therapeutic composition for interstitial pneumonia containing 10 to 100 mg of lecithinized superoxide dismutase represented by the following general formula (I):

SOD'(Q-B)ₘ (I)

(wherein, SOD' is a residue of superoxide dismutase; Q is a chemical crosslink; B is a residue of lysolecithin having the hydrogen atom of the hydroxyl group at position 2 of its glycerol moiety removed; and m is the average number of lysolecithin molecules bound to one molecule of the superoxide dismutase and is an integer of 1 or greater), and sucrose to give it a stable form suitable for intravenous administration.

Preferably, the present invention is the therapeutic composition for interstitial pneumonia, containing 40 to 80 mg of the lecithinized superoxide dismutase of the formula (I) and being provided in the form suitable for injection or intravenous drip infusion.

More preferably, the present invention is the therapeutic composition for interstitial pneumonia, being co-administered with a steroid being any of prednisolone and methylprednisolone.

Specifically, the present invention is the therapeutic composition for interstitial pneumonia wherein Q in the lecithinized superoxide dismutase of the formula (I) is represented by -C(O)-(CH₂)ₙ-C(O)- (wherein n is an integer of 2 or greater).

More specifically, the present invention is the therapeutic composition for interstitial pneumonia wherein SOD' is a residue of human superoxide dismutase, and even more specifically, SOD' is a residue of modified superoxide dismutase in which the amino acid at position 111 of an amino acid sequence of the human superoxide dismutase is modified to S-(2-hydroxyethylthio)cysteine.

Most specifically, the present invention is the therapeutic composition for interstitial pneumonia wherein the superoxide dismutase contains copper and zinc at its active center.

As used herein, the term "interstitial pneumonia" includes idiopathic interstitial pneumonias. In other words, the term includes those diseases that are classified into the following distinct clinicopathological entities: idiopathic pneumatic fibrosis (IPF), nonspecific interstitial pneumonia (NSIP), acute interstitial pneumonia (AIP), cryptogenic organizing pneumonia (COP), respiratory bronchiolitis-associated interstitial lung disease (RB-ILD), desquamative interstitial pneumonia (DIP) and lymphocytic interstitial pneumonia (LIP).
Among these interstitial pneumonias are those associated with the use of certain drugs, collagen diseases, severe acute respiratory syndrome (SARS) or new influenza viruses.

### EFFECTS OF THE INVENTION

As described above, interstitial pneumonias are often caused by collagen diseases, severe acute respiratory syndrome (SARS) or new influenza viruses, or the use of particular types of drugs, such as anticancer drugs, Chinese herbal medicines, antiallergic drugs, antibiotics and interferons and metabolites thereof. In each case, superoxide anions and other active oxygen species, as well as iron complexes, play a key role in the induction of cytotoxic effects or allergy reactions following the onset of interstitial pneumonia. The therapeutic composition of the present invention can effectively prevent such induction and, as a consequence, effectively treat interstitial pneumonias by using SOD or similar enzymes to eliminate the active oxygen species. The therapeutic composition of the present invention is also effective in the treatment of interstitial pneumonias that occur as a side effect of anticancer drug treatment.
Since no effective cure has thus far existed for interstitial pneumonia, the present invention, which can treat the disease by administration of specific PC-SOD, is of significant medical importance.
Furthermore, PC-SOD for use in the present invention has higher affinity for the cell membrane than conventional SODs and, therefore, has higher ability to eliminate superoxide anions in the affected regions. In addition, the presence of the sugar component, in particular sucrose, as a stabilizing agent can increase the stability of PC-SOD itself, so that SOD can provide prolonged effect to treat interstitial pneumonia despite its short half-life. This advantage makes the therapeutic composition for interstitial pneumonia particularly valuable.

### BEST MODE FOR CARRYING OUT THE INVENTION

The term "lecithin" as in the lecithinized superoxide dismutase (PC-SOD) used in the therapeutic composition for interstitial pneumonia of the present invention refers to common lecithin, also known as phosphatidylcholine. The term "lysolecithin" refers to a compound in which a single fatty acid molecule at position 2 of the glycerol moiety of lecithin has been substituted for a hydroxyl group.

In general, PC-SOD for use in the present invention can be obtained by binding one or more molecules of a lecithin derivative to SOD. The lecithin derivative is obtained by binding a chemical crosslinking agent to the hydroxyl group at position 2 of lysolecithin. The PC-SOD can be represented by the following formula (I):

SOD'(Q-B)ₘ (I)

wherein SOD' is a residue of superoxide dismutase; Q is a chemical crosslink; B is a residue of lysolecithin having the hydrogen atom of the hydroxyl group at position 2 of its glycerol moiety removed; and m is the average number of lysolecithin molecules bound to one molecule of the superoxide dismutase and is an integer of 1 or greater.

The SOD' used may be any of a wide range of SOD residues derived from various plants, animals or microorganisms as long as such a residue has the intended function of decomposing active oxygen (O₂⁻) in the living body. It is preferred, however, to minimize the antigenicity of the SOD residue to make it suitable for use as a pharmaceutical product. To this end, the SOD residue for use as the SOD' is preferably selected depending on the subject to which the therapeutic composition for interstitial pneumonia of the present invention is administered.

For example, SOD residues derived from humans are preferably used in order to minimize antigenicity in the living body because the therapeutic composition for interstitial pneumonia of the present invention is administered to actual human patients with interstitial pneumonia. Therefore, human SODs are preferably used in the therapeutic composition for interstitial pneumonia of the present invention because of their low antigenicity.

A particularly preferred human SOD is human Cu/Zn SOD (human SOD containing copper and zinc at the active center) since the SOD is expressed at high levels in cells and can be produced in large quantities by using the already established production techniques based on genetic engineering.

The human Cu/Zn SOD may be any type of human Cu/Zn SODs, including natural human Cu/Zn SODs prepared from human tissue or cultured human cells; human Cu/Zn SODs prepared by genetic engineering techniques; recombinant human Cu/Zn SODs having an amino acid sequence substantially identical to that of natural Cu/Zn SOD; and SODs obtained by deletion, addition, substitution or chemical modification of some of the amino acids in the amino acid sequence of the above-described human Cu/Zn SODs.

Of these human Cu/Zn SODs, a human Cu/Zn SOD in which the amino acid (cysteine: Cys) at position 111 of the amino acid sequence of natural human Cu/Zn SOD has been modified to S-(2-hydroxyethylthio)cysteine is particularly preferred. Such a human Cu/Zn SOD is described in detail in Japanese Patent Application Laid-Open No. Hei 9-117279 and can be obtained by the process described therein.
The preparation technique for human Cu/Zn SODs described in Japanese Patent Application Laid-Open No. Hei 9-117279 is incorporated herein by reference. These human Cu/Zn SODs may be used to obtain the PC-SODs for use in the present invention.

In the PC-SOD of the formula (I) for use in the present invention, "the residue of lysolecithin having the hydrogen atom of the hydroxyl group at position 2 of its glycerol moiety removed" indicated by B is specifically represented by the following formula (II):

-O-CH(CH₂OR)[CH₂OP(O)(O⁻)(OCH₂CH₂N⁺(CH₃)₃)] (II)

wherein R is a fatty acid residue (acyl group).

The fatty acid residue (acyl group) indicated by R is preferably a saturated or unsaturated fatty acid residue having 10 to 28 carbon atoms, more preferably a myristoyl group, a palmitoyl group, a stearoyl group, an icosanoyl group, a docosanoyl group or other saturated fatty acid residues having 14 to 22 carbon atoms, and particularly preferably a palmitoyl group, a saturated fatty acid residue having 16 carbon atoms.

The chemical crosslink indicated by Q in the general formula (I) may be any crosslink that can crosslink between SOD and lecithin to form a chemical bond (covalent bond). A particularly preferred chemical crosslink is the one provided by the following residue: - C(O)-(CH₂)ₙ-C(O)- (wherein n is an integer of 2 or greater). This residue is obtained by removing the hydroxyl group at each end of a straight-chained dicarboxylic acid represented by the following formula: HO-C(O)-(CH₂)ₙ-C(O)-OH, or an anhydride, an ester or a halogenated product thereof (In case of the anhydride, ester or halogenated product, the moieties corresponding to the end hydroxyl groups are removed).

When Q in the general formula (I) is the above-described straight-chained dicarboxylic acid residue, one end of Q is bound to the oxygen atom resulting from the hydroxyl group of the lysolecithin residue of the above-described formula (II) via an ester linkage, whereas the other end of Q having one end bound to the lysolecithin residue via the ester linkage is directly bound to the amino group of the SOD via an amide linkage or other similar linkages.
n in the above-described chemical crosslinking residue is an integer of 2 or greater, and preferably an integer of 2 to 10.

m in the formula (I) is the average number of lysolecithin molecules bound to one molecule of SOD. Therefore, m is an integer of 1 or greater, preferably an integer of 1 to 12, and in particular an integer of 4.

The method for producing PC-SOD for use in the present invention or specifically, the method for binding the lecithin derivative to the SOD, or preferably human Cu/Zn SOD, may be based on the technique described in Japanese Patent Application Laid-Open No. Hei 9-117279.

The PC-SOD preferably has a chemical structure as schematically shown below.

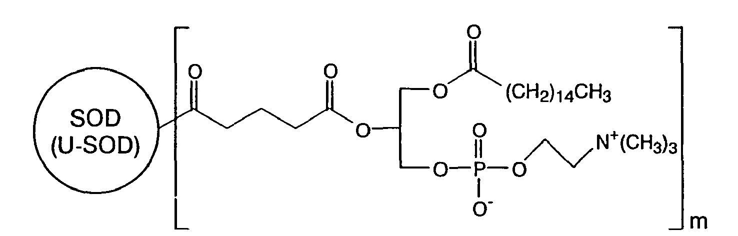

wherein m is the number of the lecithin derivatives bound to one another.

Specifically, this PC-SOD is obtained by covalently binding four molecules (average) of a lecithin derivative to a free amino group of a human Cu/Zn SOD, which can be prepared by a gene recombination technique using E. coli host cells.

Preferably, the PC-SOD for use in the therapeutic composition for interstitial pneumonia of the present invention is purified to a pharmaceutically acceptable degree and is substantially free of any pharmaceutically unacceptable materials. For example, the PC-SOD may be provided as a product that has been purified preferably to a specific SOD activity of 2,500 U/mg or higher, and more preferably to a specific SOD activity of 3,000 U/mg or higher.
In the present invention, 1 U (unit) of PC-SOD corresponds to the amount of the enzyme required for 50% inhibition of nitro blue tetrazolium (NBT) reduction as measured at pH7.8, 30°C, using the technique described in J. Biol. Chem., vol.244, No.22 6049-6055 (1969).

The therapeutic composition for interstitial pneumonia provided by the present invention, which contains the PC-SOD as an active ingredient, preferably contains a stabilizing agent together with the PC-SOD. The stabilizing agent may be a sugar component. Although such a sugar component may be any sugar component intended for pharmaceutical use, sucrose is particularly preferred. Thus, the therapeutic composition for interstitial pneumonia provided by the present invention is most preferred when it contains PC-SOD together with sucrose. Sucrose is preferably purified to a pharmaceutically acceptable degree preferably by treating it with active carbon. When used in combination with PC-SOD, such sucrose serves to prevent the activity of PC-SOD from decreasing during long-term storage, thus providing a highly stable composition that maintains favorable properties even when freeze-dried.

Although the PC-SOD and sucrose may be used in the therapeutic composition for interstitial pneumonia of the present invention in different proportions depending on the dose of the composition, dosage form and other conditions, they are preferably used at a weight ratio of PC-SOD to sucrose of about 0.1/100 to about 80/100, and more preferably at a weight ratio of about 0.4/100 to about 60/100.

The therapeutic composition for interstitial pneumonia of the present invention may further contain other pharmaceutically active components or other components commonly used in pharmaceutical products, such as excipients, binders, lubricants, coloring agents, disintegrating agents, buffers, isotonizing agents, preservatives and soothing agents, as long as such components do not affect the activity of PC-SOD or the efficacy of the composition.

The therapeutic composition for interstitial pneumonia of the present invention may be prepared by using PC-SOD and sucrose in any pharmaceutically known process commonly used in the production of pharmaceutical products. Preferably, the PC-SOD for use in the composition of the present invention is provided in the form of a solution, a frozen product or a freeze-dried product.

The therapeutic composition for interstitial pneumonia of the present invention, which contains PC-SOD stabilized by sucrose, may be administered intravenously, preferably in the form of an injection or intravenous drip infusion. The injection or intravenous drip infusion may be provided in the form of a drip infusion preparation, a solution, a suspension, an emulsion or a solid preparation designed to be dissolved upon use. All of these preparations can be prepared according to the preparation techniques described in the General Rules for Preparations Section of Japanese Pharmacopoeia.

Although the amount of PC-SOD in the therapeutic composition for interstitial pneumonia of the present invention, as well as the dose of the composition, may differ depending on the technique used to prepare the composition, dosage form, severity of the disease, and age and body weight of patients, the composition may be administered at a clinical dose of 10 to 100 mg/adult/day (30,000 to 300,000 U), and preferably at a clinical dose of 40 to 80 mg/adult/day (120,000 to 240,000 U). The composition may be administered once a day to several times a day while it may be administered at any suitable frequency.

The therapeutic composition for interstitial pneumonia of the present invention may be used to treat a wide range of interstitial pneumonias, including, but not limited to, idiopathic interstitial pneumonias of unknown etiology, interstitial pneumonias associated with the use of drugs, such as anticancer drugs, Chinese herbal medicines, antirheumatic drugs, antibiotics and interferons, and interstitial pneumonias associated with collagen diseases, severe acute respiratory syndrome (SARS) and new influenza viruses.

### Examples

The present invention will now be described in further detail with reference to a stability test and test examples in which the therapeutic composition was administered to actual patients of interstitial pneumonia (clinical test examples).
The safety of PC-SOD of the present invention was well confirmed in animal experiments prior to administration. All patients participating in the below described clinical tests were well informed of the test procedures and gave informed consent.

### Test Example 1: Stability Test

Using PC-SOD prepared according to the technique described in Japanese Patent Application Laid-Open No. Hei 9-117279, a stability test was conducted in the following manner.
Different compounds shown in Table 1 below were added to PC-SOD (0.4 mg/vial) as stabilizing agents and the resulting mixtures were dissolved in distilled water for injection. Each solution was freeze-dried.
Each of the resulting freeze-dried products was analyzed for its physical state. Each product was also analyzed by gas chromatography (GC) for the presence of peaks corresponding to analogues.
The results are summarized in Table 1 below.
The criteria for evaluation are given below.

### 1. Evaluation of physical state

A circle indicates that no apparent change has occurred in the physical state.
A cross indicates that the physical state has changed.

### 2. Evaluation of analogues by GC

A circle indicates that no peaks corresponding to analogues have appeared.
A cross indicates that peaks corresponding to analogues have appeared.

### 3. Total evaluation

A product was determined to be "poor quality" when at least one of the results of the physical state analysis and GC analysis was rated as cross "X". Otherwise, the product was determined to be "good quality."

**[Table 1]**

| Stabilizing agents (amount added) | Physical state | Analogues detected by GC | Total rating |
|---|---|---|---|
| Mannitol (20mg) | ○ | × | Poor |
| Sorbitol (20mg) | × | ○ | Poor |
| Sucrose (20mg) | ○ | ○ | Good |
| Sucrose (10mg) | ○ | ○ | Good |
| None | × | × | Poor |

As can be seen from the results of Table 1, the physical state of the freeze-dried product of PC-SOD becomes favorable and the peaks corresponding to analogues are reduced in the gas chromatography (GC) analysis when sucrose is added as a stabilizing agent.
This contrasts with the mannitol or sorbitol stabilizing agent, which is a similar sugar component but fails to provide the desired stability. Thus, the composition of the present invention containing PC-SOD and stabilized by sucrose has been proven to be particularly favorable.

### Test Example 2: Test for storage stability

According to the formulas shown in Table 2 below, freeze-dried compositions were prepared as in Test Example 1. Each composition was stored under different conditions and was subsequently analyzed for the physical state, the SOD activity, and the presence of peaks corresponding to analogues by gas chromatography (GC).
The results are summarized in Table 2. For the physical state analysis and the GC analysis for the presence of peaks corresponding to analogues, the same evaluation criteria were used as in Test Example 1.
The SOD activity of each freeze-dried composition was measured after each storage period and shown with respect to the SOD activity of the same composition measured immediately after production (100).

**[Table 2]**

| Formulas | | After storing at 40°C for 3 months | | | After storing at 25°C for 6 months | | |
|---|---|---|---|---|---|---|---|
| PC-SOD | Stabilizing agents | Physical state | Activity | GC | Physical state | Activity | GC |
| 2mg | Sucrose 100mg | ○ | 100 | ○ | ○ | 105 | ○ |
| 2mg | Sucrose 50mg | ○ | 98 | ○ | ○ | 104 | ○ |
| 2mg | Mannitol 50mg | ○ | 95 | × | ○ | 92 | ○ |
| 2mg | Sorbitol 50mg | × | 91 | × | × | 85 | × |

As can be seen from the results of Table 2, each of the freeze-dried compositions that contained PC-SOD together with sucrose was stable during each storage period. In addition, the SOD activity did not decrease in these compositions. These observations suggest the unique advantageous characteristics of the composition of the present invention containing PC-SOD and stabilized by sucrose.

### Clinical Test Example 1: Test Example 1 for interstitial pneumonia 69-year-old female

The subject was diagnosed with idiopathic interstitial pneumonia and received a steroid pulse therapy in which the subject was administered high levels of prednisolone, a steroid hormone. Since the effect of the therapy gradually dissipated over time, the subject was continued on a regimen of 30mg prednisolone + 50mg cyclosporine.
The subject suffered progressively decreasing oxygen uptake and was given as much as 5 L/min of oxygen supply at the start of the treatment. Her shortness of breath became so severe on exertion (exertional dyspnea) that the oxygen supply reached as much as 8 L/min. At this stage, daily administration of the PC-SOD of the present invention was started at 40 mg/day by intravenous infusion.
As a result, the decreased oxygen uptake was ameliorated, as were the levels of the lactose dehydrogenase (LDH) used as a measure of cytotoxic effect, the serum marker KL-6 used as a diagnostic standard of interstitial pneumonia and C-reactive protein (CRP) indicating the severity of inflammation, although the levels showed a certain degree of variation.
Approximately one month after the start of the administration of the PC-SOD of the present invention, the normal oxygen supply and the oxygen supply on exertion were improved to 3 L/min and 6 L/min, respectively. The improvement was continued subsequently.

### Clinical Test Example 2: Test Example 2 for interstitial pneumonia 67-year-old female

The subject had underlying diseases of stomach cancer and ischemic heart disease and was diagnosed with idiopathic interstitial pneumonia.
She was receiving domiciliary oxygen therapy and given 2 L/min of oxygen. Her chief complaints were shortness of breath on exertion and nonproductive cough.
At this stage, daily administration of the PC-SOD of the present invention was started at 40 mg/day by intravenous infusion.
Two days after the start of the administration, the shortness of breath became less severe and after two weeks, the subject was seen resting without being aware of not wearing the oxygen mask despite the occasional dry cough. These signs all suggested significant improvement in the patient's activities of daily living (ADL).
The Borg scale after two weeks was improved from 8 (very strong) to 2 (weak).
The decreased lung diffusion capacity (%DL_{CO}) was improved from 32.5 before administration to 61.1 nine days after administration.

The Borg scale is used as a measure of how difficult it feels for a patient to breath during exercise test or training. Specifically, a patient is asked to rate the degree of shortness of breath on a scale of 1 to 10, as given below.

**[Table 3]**

| Borg scale | |
|---|---|
| 0 | Nothing at all |
| 0.5 | Very, very weak |
| 1.0 | Very weak |
| 2.0 | Weak |
| 3.0 | Weak+ |
| 4.0 | Somewhat strong |
| 5.0 | Strong |
| 6.0 | Strong+ |
| 7.0 | Very strong |
| 8.0 | Very strong + |
| 9.0 | Very strong++ |
| 10.0 | Very, very strong |

Med. Sci. Sports Exerc., 1982:14:377-381

### Clinical Test Example 3: Test Example 3 for interstitial pneumonia 68-year-old male

The subject had underlying diseases of cholecystitis and mediastinal emphysema and was diagnosed with idiopathic interstitial pneumonia.
The subject was diagnosed with interstitial pneumonia about 2 years ago and since then received domiciliary oxygen therapy with 5L/min oxygen supply. He had been on a regimen of 30mg prednisolone (PSL) and 100mg Sandimmune (cyclosporine) before he was admitted to a hospital when the treatment turned out to be ineffective. In hospital, he developed sepsis from cholecystitis and ADL was significantly decreased. At this stage, daily administration of the PC-SOD of the present invention was started at 40 mg/day.
Starting after the start of the administration, ADL was significantly improved to a degree that the patient could move around in the ward on a wheelchair without assistance. No significant improvement in the shortness of breath on exertion was observed by the intravenous infusion of 40mg/day PC-SOD, but the shortness of breath after rehabilitation became less severe three days after the dose of PC-SOD was increased to 80 mg/day.

### Clinical Test Example 4: Test Example 4 for interstitial pneumonia 62-year-old male

The subject had a chief complaint of dry cough and was diagnosed with idiopathic interstitial pneumonia. At this stage, daily administration of the PC-SOD of the present invention was started at 40 mg/day by intravenous infusion.
About one week after the start of the administration, the shortness of breath during walking was improved significantly. Initially, the patient had shortness of breath that was so severe that he could hardly walk to the parking lot, but his shortness of breath was improved after one week and the patient no longer experienced shortness of breath when taking a bath.
The percentage of vital capacity (%VC) as a measure of respiratory function was increased from 51.0 (before administration) to 58.9 (13 days after administration).

As described above, the therapeutic composition for interstitial pneumonia of the present invention has been proven to provide significant therapeutic effects when administered to actual patients of interstitial pneumonia.
The therapeutic composition did not cause serious side effects during the actual administration period and thus was determined to be safe for use as a practical pharmaceutical product.

### INDUSTRIAL APPLICABILITY

As set forth, the therapeutic composition for interstitial pneumonia of the present invention is characterized in that it contains specific PC-SOD as an active ingredient and it is provided in the form suitable for intravenous administration. The PC-SOD has higher affinity for the cell membrane than conventional SODs and, therefore, has higher ability to eliminate superoxide anions in the affected regions. In addition, the therapeutic composition contains sucrose together with the SOD so as to increase the stability of SOD. The presence of sucrose allows the SOD to provide prolonged effect despite its short half-life. Since the SOD acts to effectively eliminate superoxide anions and other active oxygen species that induce cytotoxic effects, the therapeutic composition of the present invention can effectively prevent such induction and, as a consequence, effectively treat interstitial pneumonias. The present invention, therefore, is of significant medical importance.

## Claims

1. A therapeutic composition for interstitial pneumonia containing
10 to 100 mg of lecithinized superoxide dismutase represented by the following general formula (I):
SOD'(Q-B)ₘ (I)
(wherein SOD' is a residue of superoxide dismutase; Q is a chemical crosslink; B is a residue of lysolecithin having a hydrogen atom of a hydroxyl group at position 2 of its glycerol moiety removed; and m is the average number of lysolecithin molecules bound to one molecule of the superoxide dismutase and is an integer of 1 or greater), and
sucrose to give it a stable form suitable for intravenous administration.

2. The therapeutic composition for interstitial pneumonia according to claim 1, containing 40 to 80 mg of the lecithinized superoxide dismutase of the formula (I).

3. The therapeutic composition for interstitial pneumonia according to claim 1, being provided in the form suitable for injection or intravenous drip infusion.

4. The therapeutic composition for interstitial pneumonia according to claim 1, being co-administered with a steroid.

5. The therapeutic composition for interstitial pneumonia according to claim 4, wherein the steroid is prednisolone or methylprednisolone.

6. The therapeutic composition for interstitial pneumonia according to claim 1, wherein m is an integer of 1 to 12.

7. The therapeutic composition for interstitial pneumonia according to claim 1, wherein Q is represented by -C(O)-(CH₂)ₙ-C(O)-(wherein n is an integer of 2 or greater).

8. The therapeutic composition for interstitial pneumonia according to claim 7, wherein n is an integer of 2 to 10.

9. The therapeutic composition for interstitial pneumonia according to claim 1, wherein SOD' is a residue of human superoxide dismutase.

10. The therapeutic composition for interstitial pneumonia according to claim 1, wherein SOD' is a residue of modified superoxide dismutase in which an amino acid at position 111 of an amino acid sequence of human superoxide dismutase is modified to S-(2-hydroxyethylthio)cysteine.

11. The therapeutic composition for interstitial pneumonia according to claim 1, wherein the superoxide dismutase contains copper and zinc at its active center.

12. The therapeutic composition for interstitial pneumonia according to claim 1, wherein the interstitial pneumonia includes idiopathic interstitial pneumonias, and includes diseases that are classified into: idiopathic pneumatic fibrosis (IPF), nonspecific interstitial pneumonia (NSIP), acute interstitial pneumonia (AIP), cryptogenic organizing pneumonia (COP), respiratory bronchiolitis-associated interstitial lung disease (RB-ILD), desquamative interstitial pneumonia (DIP) and lymphocytic interstitial pneumonia (LIP).

13. The therapeutic composition for interstitial pneumonia according to claim 1, wherein the interstitial pneumonia is caused by collagen diseases or use of a drug.

14. The therapeutic composition for interstitial pneumonia according to claim 13, wherein the drug includes an anticancer drug, a Chinese herbal medicine, an antirheumatic drug, an antibiotic and an interferon.

15. The therapeutic composition for interstitial pneumonia according to any one of claims 1 to 13, wherein the interstitial pneumonia is associated with severe acute respiratory syndrome (SARS) or new influenza viruses.
